# EUROPÄISCHE PATENTSCHRIFT

(11) **EP 4 275 768 B1**
(45) Veröffentlichungstag und Bekanntmachung des Hinweises auf die Patenterteilung: **02.10.2024**
(21) Anmeldenummer: 22172267.1
(22) Anmeldetag: 09.05.2022
(51) Int. Cl.: A63B 71/12, A41D 13/06, A43B 5/02, A61F 13/06, A41D 13/05, A63B 69/00, A43B 19/00

(54) **SPORTGERÄT MIT POSITIONIERHILFE FÜR EIN SCHUTZELEMENT**
SPORTS DEVICE WITH A POSITIONING AID FOR A PROTECTIVE ELEMENT
APPAREIL DE SPORT POURVU D'AIDE AU POSITIONNEMENT POUR UN ÉLÉMENT DE PROTECTION

(43) Veröffentlichungstag der Anmeldung: 15.11.2023
(73) Patentinhaber: Boccino Esposito, David Enea, 5706 Boniswil (CH)
(72) Erfinder: Boccino Esposito, David Enea, 5706 Boniswil (CH)
(74) Vertreter: Christ, Niko

(56) Entgegenhaltungen:
- KR-Y1- 200 363 025
- US-A1- 2009 205 097
- US-B2- 10 897 951

## Beschreibung

Die vorliegende Erfindung betrifft ein fußgetragenes Sportgerät, insbesondere für einen Fußballspieler, umfassend eine Positionierhilfe, an welcher ein Schutzelement in definierter Position befestigt ist, wobei das Schutzelement eine Keilauflage an der Positionierhilfe ausbildet, welche eine konkave Innenfläche aufweist, die sich an den Fußrücken eines die Positionierhilfe tragenden Fußes anschmiegt und der eine ebene Außenfläche gegenüberliegt, welche mit dem Fußrücken einen definierten Ballkontakt erlaubt.

Ein derartiges Sportgerät ist bereits aus der US 10,897,951 B2 vorbekannt. Weiterer Stand der Technik ergibt sich aus der DE 19 47 427 U, der US 2,796,684 A, der US 2014/0259260 A1, der KR200363025 Y1 und der US 2009/205097 A1.

Ferner sind vergleichbare Sportgeräte aus dem Stand der Technik vorbekannt. Hierbei handelt es sich um Bandagen, Manschetten, Socken oder Strümpfe, zum Beispiel für Fußballspieler, die einen Schienbeinschutz unverrückbar am Schienbein halten. Ein solcher Schienbeinschutz ist dabei aus einem Kunststoff, Schaumeinlagen und/oder aus schockabsorbierenden Textillagen gefertigt und soll Beinareale, die häufigen Stößen und Schlägen ausgesetzt sind, schützen, indem der Schienbeinschutz den Großteil der Kraft absorbiert oder ableitet.

Ähnliche Ausführungsformen sind auch aus dem Kampfsport bekannt, wobei hier der Schutz sich nicht nur an den Schienbeinen befindet, sondern auch auf den Fußrücken erstreckt sein kann. Das Fußrückensegment kann dabei den ganzen Fußrücken bedecken. Ein solcher Fußrückenschutz hat primär die Aufgabe, die Kraft von Schlägen, die mit dem Fußrücken geübt werden, gedämpft an den Fuß weiterzugeben.

Allgemein dienen solche Sportgeräte dem Zweck, die Kraft von Schlägen und Stößen, die auf ein Körperteil oder mit einem Körperteil ausgeübt werden, teilweise zu absorbieren und abzuleiten. Durch den Einsatz eines solchen Schutzes wird das Verletzungsrisiko gesenkt.

Jedoch können derartige Einlagen neben der reinen Schutzfunktion auch weitere Funktionen, wie etwa eine definierte Formung, erfüllen. So ist es etwa aus Handschützern für das Inline skaten bekannt, Formteile einzusetzen, welche einen Aufprall nicht nur abmildern, sondern auch eine definierte Auflagefläche und eine Sollbruchfläche definieren können.

Vor diesem Hintergrund liegt der vorliegenden Erfindung die Aufgabe zu Grunde, ein fußgetragenes Sportgerät zu schaffen, welches einerseits einen Schutz für den Fußrücken bereithält, andererseits aber eine nützliche, definierte Form einnimmt.

Gelöst wird diese Aufgabe durch ein fußgetragenes Sportgerät gemäß den Merkmalen des unabhängigen Anspruchs 1. Sinnvolle Ausgestaltungen eines solchen Sportgeräts können den sich anschließenden abhängigen Ansprüchen entnommen werden.

Erfindungsgemäß ist ein fußgetragenes Sportgerät, insbesondere für einen Fußballspieler, vorgesehen, umfassend eine Positionierhilfe, an welcher ein Schutzelement in definierter Position befestigt ist, wobei das Schutzelement eine Keilauflage an der Positionierhilfe ausbildet, welche eine konkave Innenfläche aufweist, die sich an den Fußrücken eines die Positionierhilfe tragenden Fußes anschmiegt und der eine ebene Außenfläche gegenüberliegt, welche mit dem Fußrücken einen definierten Ballkontakt erlaubt.

Das Schutzelement nimmt daher in der Erfindung eine Doppelfunktion wahr. Zum Einen schützt es den Fußrücken vor Verletzungen, zum anderen bildet es aber auch eine Fläche aus, mit der eine verbesserte Ballkontrolle ermöglicht ist. Da das Schutzelement in einer keilförmigen Ausgestaltung vorliegt, wird die ebene Oberfläche, die sich an den Fußrücken anschließt, dazu dienen, die Kontaktfläche des Fußrückens mit dem Ball in der Ausrichtung so anzupassen, dass die Kontaktfläche direkt nach vorne weist. Es fällt dadurch leichter, den Ball kontrolliert in die Laufrichtung zu spielen, ohne den Fuß zu verdrehen. Die zur Verfügung stehende Fläche, die gerade nach vorne weist, wird mit dem erfindungsgemäßen Schutzelement vergrößert. Mit einer derart abgeflachten Oberfläche kann der Ball besser gelenkt werden als mit der Kurvatur des Fußrückens.

Um die Flexibilität des Fußes beim Laufen nicht einzuschränken, wird die Keilauflage, die als Schutzelement dienen soll, mittels einer Positionierhilfe derart am Fußrücken angebracht, dass sie nicht verrutschen kann. Zusätzlich darf die Keilauflage beim Abrollvorgang des Fußes nicht im Weg sein, da sonst die Sprintgeschwindigkeit abnimmt. Dabei weist die Keilauflage eine konkave Unterseite auf, die sich ergonomisch an den Fußrücken anlegt, um den Druck gleichmäßig in die Fläche des Fußrückens einzuleiten und die ebene Außenfläche möglichst vollständig abzustützen.

Weiter ist es vorgesehen, dass an der Keilauflage ein Ausläufer ausgebildet ist, welcher sich entlang der Fußaußenkante eines die Positionierhilfe tragenden Fußes in Richtung von dessen Ferse erstreckt. Hierdurch wird das Areal vor dem häufig auftretenden unerwünschten Kontakt mit anderen Fußballspielern geschützt, wie es im Laufe des Spiels oft der Fall ist. Dies führt häufig zu Verletzungen, da die Stollen des Gegenspielers mit großer Wucht auf die ungeschützte Fußaußenseite treffen können.

Jedoch kann es auch von Vorteil sein, wenn die Positionierhilfe eine Socke, insbesondere eine Sportsocke, ist. Die Verbindung der Keilauflage mit einer Socke stellt eine anwendungsflexiblere und günstigere Variante der Erfindung dar. In dieser Variante ist der Anwender nicht darauf festgelegt, das Schutzelement jedes Mal anzuwenden. Der Benutzer kann von mal zu mal entscheiden, ob er das Schutzelement einsetzen möchte oder nicht.

Weiter ist es vorgesehen, dass die Positionierhilfe eine Socke mit Aussparungen an Ferse und/oder an den Zehen ist. Diese Ausführungsform bietet zusätzlich die Möglichkeit, die Socken ebenfalls unabhängig von der Positionierhilfe zu wählen, indem sie das umgebende und wärmende Material der Positionierhilfe auf ein Minimum beschränkt, ohne dabei auf die geforderten Eigenschaften verzichten zu müssen. Zusätzlich fixiert eine solche Positionierhilfe mittels der genannten Aussparungen an Ferse und Zehen die Keilauflage deutlich besser am Fuß, da die Positionierhilfe hierdurch selbst noch einmal definierter am Fuß anliegt. Das bietet den weiteren Vorteil, dass die Positionierhilfe auch bei extremer Mobilität des Fußes, wie sie etwa während eines Sprints auftritt, sich mit dem Fuß bewegt, als wäre sie ein Teil von ihm. Hierdurch werden Relativbewegungen zwischen dem Fuß und der Keilauflage deutlich minimiert.

Es hat sich weiterhin als vorteilhaft erwiesen, wenn die Keilauflage mit der Positionierhilfe unlösbar verbunden ist, insbesondere durch Kleben, Schweißen, Nähen oder Zwischenfließen in das Material der Positionierhilfe. Es ist wesentlich, dass die Keilauflage eine fixe Position am Fußrücken einnehmen kann. Dies wird zwar schon von der ergonomischen, konkaven Unterseite zum Teil bewerkstelligt, jedoch muss auch während des Bewegungsablaufs beim Einsatz und insbesondere während des Ballkontakts gewährleistet sein, dass die Keilauflage nicht aus ihrer Position rutschen kann. Wenn die Keilauflage unlösbar mit dem Material der Positionierhilfe verbunden ist, dann kann sie auch dann in Position gehalten werden, wenn Scherkräfte durch einen schräg aufgenommenen Ball auf den Fuß einwirken.

In der oben genannten Ausführung kann es von Vorteil sein, wenn die Positionierhilfe aus biegeschlaffem und elastischem Material oder Gewebe, insbesondere aus einer Mischung, die Nylon enthält, besteht. Durch eine textile Ausfertigung der Positionierhilfe kann auf die erforderliche Flexibilität des Fußes, wie sie etwa beim Fußballspiel notwendig ist, Rücksicht genommen werden. Da Nylon oder Mischungen, die Nylon enthalten, Dank des geringen Gewichts, der Strapazierfähigkeit und der Atmungsaktivität zusehends im Sport Anwendung finden, bietet es sich an, diese Vorteile des Materials auch für die Positionierhilfe zu nutzen.

Es hat sich auch als vorteilhaft erwiesen, wenn die Keilauflage aus einem formstabilen, schockabsorbierenden Material, vorzugsweise aus einem Material auf Silikonbasis, besteht. Im Falle, dass die Keilauflage aus einem Material auf Silikonbasis gefertigt ist, kann sie mehrere vorteilhafte Eigenschaften in sich vereinen. Sollte direkter Kontakt mit der Haut bestehen, kann sich zum einen die Keilauflage hautfreundlich anschmiegen, zum anderen kann das Material bei der Produktion leicht in Form gebracht werden. Des Weiteren kann das Material spontane Schockeinwirkungen gut absorbieren ohne dass es hierdurch zu vorschneller Materialermüdung kommt.

Die vorliegend beschriebene Erfindung wird im Folgenden anhand eines Ausführungsbeispiels näher erläutert.

Es zeigen:
- Figur 1: ein rechter Fuß mit einer Positionierhilfe und einer daran angeordneten Keilauflage in perspektivischer Darstellung,
- Figur 2: ein linker Fuß mit einer Positionierhilfe und einer daran angeordneten Keilauflage in frontaler Darstellung, sowie
- Figur 3: der Fuß gemäß Figur 2 in perspektivischer Darstellung von schräg vorne.

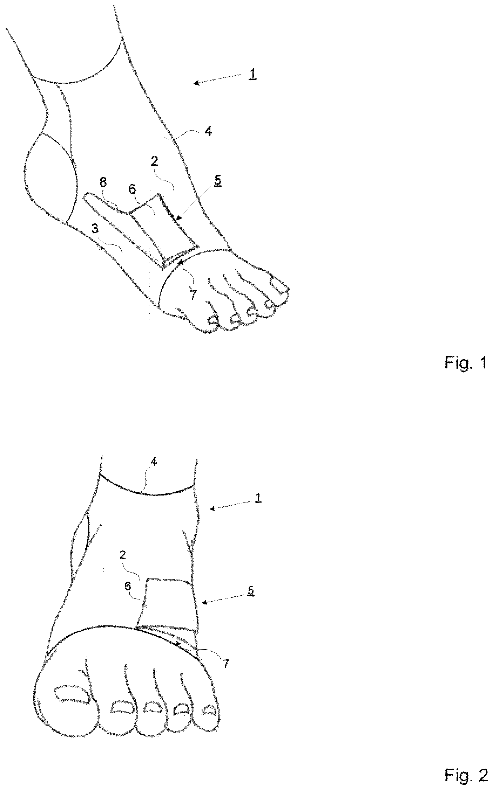

Figur 1 zeigt ein Sportgerät, welches mithilfe einer Positionierhilfe 4 am Fuß 1 getragen wird. Das Sportgerät weist hierbei eine Keilauflage 5 auf, welche an ihrer von dem Fuß 1 abgewandten Seite eine ebene Außenfläche ausbildet. Diese Außenfläche stellt eine definierte Anprallfläche für einen Ball dar und sorgt dafür, dass der Ball trotz der gebogenen Form des Fußrückens definiert in eine gewünschte Richtung gelenkt werden kann. Die eng anliegende Positionierhilfe 4 fixiert hierbei die Keilauflage 5 derart, dass eine Außenfläche 6 der Keilauflage 5 an ihrem spitz zulaufenden Ende an einem Fußrücken 2 zum Liegen kommt. Die konkave Innenfläche 7 schmiegt sich an den Fuß 1 an. Ein Ausläufer 8 bildet eine dritte Seite der Keilauflage 5 und erstreckt sich in Richtung Ferse. Durch die Verlängerung des Ausläufers 8 in Richtung der Ferse wird eine Fußaußenkante 3 mit einem Schutzelement versehen. Die Positionierhilfe 4 stabilisiert die Keilauflage 5 in ihrer Position auf dem Fuß 1 während der typischen Bewegungsabläufe eines Fußballspiels. Dies bedeutet, dass die Keilauflage 5 während des Laufens, insbesondere bei Ballkontakt, nicht verrutschen darf, da sie ansonsten den Fußballspieler in seiner Bewegung einschränkt und durch ein Verdrehen auch die Ballkontrolle erschwert. Wenn die Positionierhilfe 4 aus einem leichten, flexiblen Stoff hergestellt ist, kann sie sich an die Anatomie des Fußes optimal anlegen und das Verrutschen der Keilauflage 5 verhindern. Hierbei ist vor allem die Ausführung einer Socke mit Aussparungen an den Zehen und der Ferse besonders als stabil anzusehen, da die Aussparungen ihrerseits die Lage der Positionierhilfe 4 auf dem Fuß noch einmal eindeutig fixieren.

Figur 2 zeigt eine Frontalansicht auf die Positionierhilfe 4 und der darauf angebrachten Keilauflage 5. Hierbei wird deutlich, dass die konkave Innenfläche 7 sich an den Fußrücken 2 anschmiegt, um den bestmöglichen Halt zu gewährleisten. Ein möglichst passgenaues Anliegen der Keilauflage 4 ist notwendig, damit auch die Stöße, die während des Kontakts mit einem Ball oder einem weiteren Spieler auftreten, gut über die gesamte Kontaktfläche abgeleitet werden können. Des Weiteren ist die Keilauflage 5 derart geformt, dass sie nahe dem Zentrum des Fußrückens 2 beginnt und sich zur Fußaußenkante 3 hin keilförmig erweitert. Die nach außen zunehmende Mächtigkeit der Keilauflage 5 hilft auch bei der Schockabsorption, hat aber auch den Vorteil, dass die ebene Außenfläche 6 das seitliche Abfallen des Fußrückens kompensiert. Durch das Vorhandensein dieser größeren ebenen Außenfläche 6 auf dem Fußrücken 2 kann der Ball mit einer höheren Präzision gespielt werden.

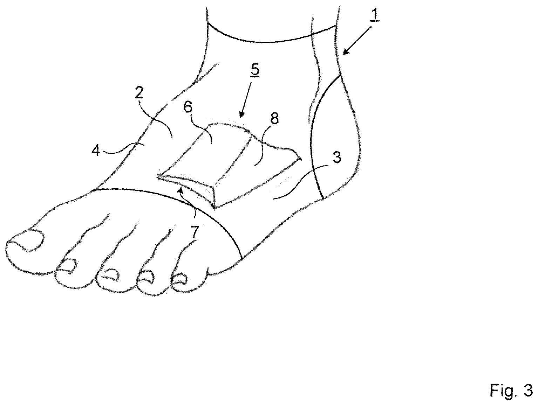

Figur 3 zeigt das Sportgerät von der Fußaußenkante 3 her, wobei deutlich wird, dass der Ausläufer 8 sich an die Fußaußenkante 3 anschmiegt. Bei der Ausformung der Keilauflage 5 ist es wichtig, dass das Material eine gewisse Flexibilität aufweist und Krafteinwirkungen gut absorbieren kann, ohne spröde auf diese zu reagieren. Der Ausläufer 8 wird in seiner Erstreckung Richtung Ferse immer dünner, um möglichst viel von der Fußaußenkante 3 abzudecken, jedoch dabei die Fußaußenkante 3 nicht unnötig zu versteifen, da ansonsten das Abrollen des Fußes 1 behindert wird. Dennoch muss die relativ dünne Materialschicht Schlägen und Stößen standhalten und diese ableiten. Die Fixierung der Keilauflage 5 mittels einer Positionierhilfe 4 am Fuß 1 sorgt dafür, dass die Keilauflage 5 sich bei allen Bewegungen mit dem Fuß 1 mitbewegt. Durch das Minimieren jeglicher Bewegung der Keilauflage 5 relativ zum Fuß 1, kann eine Verletzung des Fußes 1 durch die Keilauflage 5, vermieden werden.

Vorstehend beschrieben ist somit ein fußgetragenes Sportgerät, welches einerseits einen Schutz für den Fußrücken bereithält, andererseits aber eine nützliche, definierte Form einnimmt.

### BEZUGSZEICHENLISTE

- 1: Fuß
- 2: Fußrücken
- 3: Fußaußenkante
- 4: Positionierhilfe
- 5: Keilauflage
- 6: Außenfläche
- 7: Innenfläche
- 8: Ausläufer

## Patentansprüche

1. Fußgetragenes Sportgerät, insbesondere für einen Fußballspieler, umfassend eine Positionierhilfe (4), an welcher ein Schutzelement in definierter Position befestigt ist, wobei das Schutzelement eine Keilauflage (5) an der Positionierhilfe (4) ausbildet, welche eine konkave Innenfläche (7) aufweist, die sich an den Fußrücken (2) eines die Positionierhilfe (4) tragenden Fußes (1) anschmiegt und der eine ebene Außenfläche (6) gegenüberliegt, welche mit dem Fußrücken (2) einen definierten Ballkontakt erlaubt, wobei die Positionierhilfe (4) eine Socke, insbesondere eine Sportsocke, ist und die Keilauflage (5) mit der Positionierhilfe (4) unlösbar verbunden ist, insbesondere durch Kleben, Schweißen, Nähen oder Zwischenfließen in das Material der Positionierhilfe (4),wobei die Keilauflage (5) aus einem formstabilen, schockabsorbierenden Material, vorzugsweise aus einem Material auf Silikonbasis, besteht und an der Keilauflage (5) ein Ausläufer (8) ausgebildet ist, welcher sich entlang der Fußaußenkante (3) eines die Positionierhilfe (4) tragenden Fußes (1) in Richtung von dessen Ferse erstreckt.

2. Sportgerät gemäß Anspruch 1, **dadurch gekennzeichnet, dass** die Positionierhilfe (4) eine Socke mit Aussparungen an Ferse und/oder an den Zehen ist.

3. Sportgerät gemäß wenigstens einem der vorhergehenden Ansprüche, **dadurch gekennzeichnet, dass** die Positionierhilfe (4) aus biegeschlaffem und elastischem Material oder Gewebe, insbesondere aus einer Nylon enthaltenden Mischung, besteht.

## Claims

1. Foot-worn sports equipment, in particular for a football player, comprising a positioning aid (4) to which a protective element is attached in a defined position, wherein the protective element forms a wedge support (5) on the positioning aid (4), which wedge support has a concave inner surface (7) which conforms to the instep (2) of a foot (1) wearing the positioning aid (4) and which is opposite a planar outer surface (6) which allows defined ball contact with the instep (2), wherein the positioning aid (4) is a sock, in particular a sports sock, and the wedge support (5) is permanently connected to the positioning aid (4), in particular by gluing, welding, sewing or flowing into the material of the positioning aid (4),wherein the wedge support (5) consists of a dimensionally stable, shock-absorbing material, preferably of a silicone-based material, and an extension (8) is formed on the wedge support (5), which extends along the foot outer edge(3) of a foot (1) wearing the positioning aid (4) in the direction of its heel.

2. Sports equipment according to claim 1, **characterized in that** the positioning aid (4) is a sock having openings on the heel and/or on the toes.

3. Sports equipment according to at least one of the preceding claims, **characterized in that** the positioning aid (4) consists of flexible and elastic material or fabric, in particular of a mixture containing nylon.

## Revendications

1. Appareil pour le sport porté par le pied, en particulier pour un joueur de football, comprenant un moyen auxiliaire de positionnement (4) sur lequel est fixé un élément de protection dans une position définie, dans lequel l'élément de protection forme un support en forme de coin (5) sur le moyen auxiliaire de positionnement (4), lequel support présente une surface intérieure concave (7), laquelle s'adapte au dos du pied (2) d'un pied (1) portant le moyen auxiliaire de positionnement (4) et est opposée à une surface extérieure plane (6) qui permet un contact défini avec le ballon avec le dos du pied (2), dans lequel le moyen auxiliaire de positionnement (4) est une chaussette, en particulier une chaussette pour le sport, et le support en forme de coin (5) est relié de manière inamovible au moyen auxiliaire de positionnement (4), en particulier par collage, soudage, couture ou coulée intermédiaire dans le matériau du moyen auxiliaire de positionnement (4), dans lequel le support en forme de coin (5) est constitué d'un matériau indéformable et absorbant les chocs, en particulier d'un matériau à base de silicone, et un prolongement (8) est réalisé sur le support en forme de coin (5), lequel prolongement s'étend le long du bord extérieur de pied (3) d'un pied (1) portant le moyen auxiliaire de positionnement (4) en direction de son talon.

2. Appareil pour le sport selon la revendication 1, **caractérisé en ce que** le moyen auxiliaire de positionnement (4) est une chaussette comportant des évidements au niveau du talon et/ou au niveau des orteils.

3. Appareil pour le sport selon au moins l'une des revendications précédentes, **caractérisé en ce que** le moyen auxiliaire de positionnement (4) est constitué d'un matériau ou d'un tissu souple et élastique, en particulier d'un mélange contenant du nylon.
